# EUROPÄISCHE PATENTSCHRIFT

(11) **EP 1 155 678 B1**
(45) Veröffentlichungstag und Bekanntmachung des Hinweises auf die Patenterteilung: **01.03.2006**
(21) Anmeldenummer: 01111813.0
(22) Anmeldetag: 16.05.2001
(51) Int. Cl.: A61Q 5/04, A61K 8/34

(54) **Mittel zur dauerhaften Verformung von menschlichen Haaren**
Composition for permanent deformation of hair
Composition pour la déformation permanente des cheveux

(30) Priorität: 18.05.2000 DE 10024671
(43) Veröffentlichungstag der Anmeldung: 21.11.2001
(73) Patentinhaber: KPSS-Kao Professional Salon Services GmbH, 64297 Darmstadt (DE)
(72) Erfinder: Rose, Burkhard, 64297 Darmstadt (DE); Schneider, Jörg, 64347 Griesheim (DE); Wood, Jonathan, Dr., 69469 Weinheim (DE)

(56) Entgegenhaltungen:
- EP-A- 0 658 338
- EP-A- 0 666 073
- WO-A-99/06013
- DE-C- 19 714 162
- GB-A- 722 328
- US-A- 4 532 127
- US-A- 6 017 519
- PATENT ABSTRACTS OF JAPAN vol. 2000, no. 05, 14. September 2000 (2000-09-14) & JP 2000 044441 A (KAO CORP), 15. Februar 2000 (2000-02-15)
- DATABASE WPI Section Ch, Week 199351 Derwent Publications Ltd., London, GB; Class D21, AN 1993-408827 XP002174543 & JP 05 306212 A (KAO CORP), 19. November 1993 (1993-11-19)
- DATABASE CHEMABS [Online] CHEMICAL ABSTRACTS SERVICE, COLUMBUS, OHIO, US; HOSOKAWA, MINORU ET AL: "Effects of polyols on human hair" retrieved from STN Database accession no. 127:140171 XP002174542 & NIPPON KESHOHIN GIJUTSUSHA KAISHI (1997), 31(2), 167-175 ,

## Beschreibung

Die vorliegende Erfindung betrifft ein Mittel zum Verformen von menschlichen Haaren, d.h. ein Dauerwellmittel, das eine ausgezeichnete Wellwirkung besitzt, jedoch auf das Haar auch bei mehrfacher Anwendung keinerlei schädigende Wirkung ausübt.

Die Dauerwellung erfordert bekanntlich zwei Behandlungsschritte:
Die reduktive Spaltung der Cystein-Disulfidbrücken des Haares durch Einwirkung eines Reduktionsmittels und die anschließende Neutralisierung bzw. Fixierung durch Aufbringung eines Oxidationsmittels, wodurch die Cystein-Disulfidbrücken wiederhergestellt werden.

Das überwiegend eingesetzte Reduktionsmittel ist auch heute noch Thioglykolsäure, insbesondere als Ammoniumsalz, obwohl zahlreiche andere Thio-Verbindungen für diesen Zweck vorgeschlagen wurden, die sich jedoch in der Praxis zumeist nicht durchgesetzt haben.

Die Thioglykolat enthaltenden Zusammensetzungen werden üblicherweise bei einem pH-Wert zwischen 8 und 10, insbesondere 8,5 bis 9,5, eingesetzt, was bei wiederholter, zeitlich nahe zusammenliegender Anwendung zu Haarschädigungen führen kann.

Man hat bereits versucht, diese Nachteile durch die Schaffung sogenannter "saurer Dauerwellmittel" zu überwinden, deren Anwendungs-pH-Wert bei etwa 6,8 bis 7,8, d.h., um den Neutralpunkt, herum liegt. Der in diesen Zusammensetzungen meistbenutzte reduzierende Wirkstoff ist der Thioglykolsäuremonoglycerinester. Es hat sich jedoch gezeigt, daß diese Substanz bei manchen Benutzerinnen hautreizend, insbesondere sensibilisierend, wirkt, so daß auch diese Lösung nicht optimal ist.

Es wurde nunmehr gefunden, daß diese Probleme dadurch überwunden werden können und Dauerwellmittel, die nicht haarschädigend sind, jedoch gleichwohl eine gute Wellwirkung aufweisen, erhalten werden, wenn man ein Mittel einsetzt, das mindestens eine reduzierend wirkende Thioverbindung, vorzugsweise Thioglykolsäure und/oder Thiomilchsäure bzw. deren Salze, sowie ein Kombination aus
a) 0,25 bis 7,5 Gew.-% Ethylalkohol, n-Propylalkohol und/oder Isopropylalkohol;
b) 0,25 bis 7,5 Gew.-% mindestens einer Komponente aus der Gruppe Pentandiole, Hexandiole und/oder Methylpentandiole; und
c) 0,25 bis 7,5 Gew.-% mindestens einer Komponente aus der Gruppe 1-Methoxypropanol(-2), 1-Ethoxypropanol(-2) und/oder Diethylenglykolmonomethyl- bzw. -monoethylether,
jeweils berechnet auf die Gesamtzusammensetzung des gebrauchsfertigen Mittels, enthält.

Aus der EP 666 073 A2 sind bereits Dauerwellmittel bekannt, die ähnliche Lösungsmittelgemische enthalten, wobei der Bestandteil b) jedoch aus mindestens einem Bestandteil der Gruppe 1,2-Propandiol, 1,2-Butandiol, 1,3-Butandiol, Glycerin und/oder Dipropylenglykolmonomethylether besteht.

DE 197 14 162 C1, EP 658 338 A1 und Hosokawa et al (Nippon Keshohin Gijutsusha Kaishi 31(2), 167 - 175, 1997) offenbaren Mittel enthaltend Pentandiols zur dauerhaften Verformung von menschlichen Haaren die bei guter Verformungswirksamkeit gleichzeitig eine weniger haarschädigende Wirkung auf das Haar haben.

Es wurde jedoch überraschenderweise festgestellt, daß deren erfindungsgemäßer durch Pentan-, Methylpentan- und/oder Hexandiole eine nochmals verbesserte Wellwirksamkeit ergibt und auch die dermatologische Verträglichkeit der Gesamtzusammensetzung erhöht.

Gemäß einer bevorzugten Ausführungsform der Erfindung weist die gebrauchsfertige, zum Aufbringen auf das Haar bestimmte Dauerwell-Zusammensetzung einen pH-Wert zwischen etwa 6,5 und 10,5, insbesondere 7,0 bis etwa 9,5 auf.

Wie bereits dargestellt, enthält das erfindungsgemäße Dauerwellmittel als bevorzugte reduzierend wirkende Thioverbindungen Thioglykolsäure und/oder 2- bzw. 3-Thiomilchsäure bzw. deren Salze, beispielsweise Diammonium- bzw. Alkalisalze.

Es können jedoch auch andere wellwirksame Thioverbindungen mitverwendet werden, beispielsweise die aus der EP-A 235 783 bekannten Thioglyceryl-C₁-C₂₀₋alkylether oder 1-Phenyl-2-mercaptoethanol sowie die in der EP-A 461 526 beschriebenen C₁-C₂₀-Alkyloxyethoxythioglycerylether.

Weitere einsetzbare Thioverbindungen sind vor allem Cystein bzw. dessen Hydrochlorid, Homocystein, Cysteamin, N-Acetylcystein, Thioglycerin, Ethandiolmonothioglykolat, 1,2-Propylenglykolmonothioglykolat (vgl. auch WO-A 93/1791), 1-3-Propandiolmonothioglykolat bzw. das daraus resultierende Isomerengemisch, 1,3-Butandiol- und 1,4-Butandiolmonothioglykolat bzw. deren Isomerengemische, Ethandiolmonothiolactat, 1,2-Propandiol- und 1,3 Propandiolmonothiolactat und deren Isomerengemische, 1,3-Butandiol- und 1,4-Butandiolmonothiolactat und deren Isomerengemische, Polyethylenglykol- wie Di-, Tri- und Tetraethylenglykolmonothioglykolate und -monothiolactate, Polypropylenglykol- wie Di-, Tri- und Tetrapropylenglykolmonothiolactate und -monothioglycolate, Glycerinmonothiolactat und weitere Thiosäuren und deren Ester sowie Gemische derselben.

Der Gesamtgehalt an Reduktionsmittein in den erfindungsgemäßen Zusammensetzungen beträgt üblicherweise 2,5 bis etwa 15 Gew.-%, berechnet auf freie Thioglykolsäure als Bezugssubstanz.

Auch die Verwendung anorganischer Sulfite, insbesondere Alkalisulfite und Alkalihydrogensulfite beispielsweise Natriumbisulfit, ist grundsätzlich möglich.

Die Reduktionsmittel enthaltenden Dauerwellpräparate können, falls erforderlich, einen Gehalt an Alkalisierungsmitteln aufweisen. Die Menge ist anhängig vom reduzierenden Wirkstoff und dem angestrebten pH-Wert der Zusammensetzung. Vorzugsweise enthält die Reduktionsmittel-Zusammensetzung etwa 0,1 bis etwa 10, insbesondere etwa 0,5 bis etwa 2,5 Gew.-% desselben.

Bevorzugte Alkalisierungsmittel im Rahmen der Erfindung sind Ammoniumcarbamat, Ammoniak und/oder Ammonium(bi)carbonat. Es wird, wie gesagt, die Einstellung eines pH-Wertes im Bereich zwischen etwa 6,5 und etwa 10,5, vorzugsweise etwa 7 bis 9,5, angestrebt.

Das Alkalisierungsmittel kann gemeinsam mit der wellwirksamen Thioverbindung in einer Phase vorliegen; es kann jedoch, aus Kompatibilitäts- und Stabilitätsgründen, auch sinnvoll sein, beide Stoffe in jeweils bis zur Anwendung getrennt gehaltener Form, beispielsweise in Zweikammerbehältern, zu lagern, die erst unmittelbar von der Applikation auf das Haar vermischt werden.

Die erfindungsgemäß zum Einsatz kommenden Dauerwellmittel enthalten vorzugsweise auch Tenside. Deren Anteil liegt bei etwa 0,1 bis etwa 10, insbesondere etwa 1 bis etwa 5 Gew.-%, der das Reduktionsmittel,enthaltenden Zusammensetzung.

Sowohl bei den in den Reduktionsmittel-Zusammensetzungen als auch bei den in den Fixiermitten eingesetzten Tensiden handelt es sich vorzugsweise um die bekannten anionaktiven Produkte, die gegebenenfalls auch in Kombination mit nichtionischen Tensiden zum Einsatz gelangen.

Geeignete anionische Tenside sind besonders die bekannten Alkylethersulfate und -carbonsäuren, insbesondere in Form ihrer Alkalisalze, sowie Eiweiß-Fettsäure-Kondensate.

Geeignete nichtionische Tenside sind insbesondere C₈-C₁₈-Fettalkoholpolyglykolether, Fettsäurepolyglykolester, Fettsäurealkanolamide, Aminoxide und vor allem C₈-C₁₈-Alkylpolyglukoside.

Es können auch amphotere Tenside wie die bekannten Betaine und Amidobetaine sowie, kationaktive Tenside wie quaternäre Ammoniumverbindungen eingesetzt werden.

Die zweite essentielle erfindungsgemäße Substanz, mindestens eine Verbindung aus der Gruppe Ethylalkohol, n-Propylalkohol und/oder Isopropylalkohol, ist in einer Menge von 0,25 bis etwa 7,5 Gew.-%, vorzugsweise etwa 0,5 bis 5 Gew.-%, insbesondere etwa 0,75 bis 2,5, jeweils berechnet auf die Gesamtzusammensetzung, enthalten. Vorzugsweise wird Isopropylalkohol eingesetzt.

Der dritte essentielle Bestandteil, mindestens eine Komponente aus der Gruppe Pentandiole, Hexandiole und/oder Methylpentandiole liegt ebenfalls in einer Menge von 0,25 bis etwa 7,5, vorzugsweise etwa 0,5 bis etwa 5 Gew.-%, berechnet auf die gebrauchsfertige Gesamtzusammensetzung, vor. Bevorzugt sind 1,2- und 1,5-Pentandiol, 1,6-Hexandiol, 3-Methyl-1,5-pentandiol und 2-Methyl-2,4-Pentandiol, vorzugsweise in einer Menge von etwa 0,5 bis etwa 3 Gew.-%.

Der letzte essentielle erfindungsgemäße Bestandteil, 1-Methoxypropanol(-2), 1-Ethoxypropanol(-2), und/oder Diethylenglykolmonomethyl- bzw. -monoethylether, ist ebenfalls in einer Menge zwischen etwa 0,25 und etwa 7,5 Gew.-%, berechnet auf die gebrauchsfertige Gesamtzusammensetzung, enthalten. Hier sind Diethylenglykolmonoethylether, auch unter dem Namen Ethoxydiglykol bzw. Ethylcarbitol bekannt, und 1-Methoxypropanol(-2) in einer Menge zwischen etwa 1 und etwa 5 Gew.-% bevorzugt.

Die erfindungsgemäßen Dauerwellmittel können alle für diesen Zweck vorgeschlagenen und eingesetzten Zusatzstoffe enthalten.

Zur Vermeidung von Wiederholungen wird hierzu auf den einschlägig bekannten Stand der Technik und dessen Zusammenfassungen, beispielsweise in "Ullmann's Encyclopaedia of Industrial Chemistry, Vol A12 (1986), S. 588 bis 591, sowie der Monographie von K. Schrader, "Grundlagen und Rezepturen der Kosmetika", 2. Auflage (1989, Hüthig Buchverlag) S. 823- bis 840, verwiesen.

Falls das Mittel in zwei bis zur Anwendung getrennten Phasen vorliegt, ist es vorteilhaft, den pH-Wert einer Phase im sauren Bereich, beispielsweise zwischen etwa 4,5 und 6,5, einzustellen.

Dies geschieht beispielsweise durch Zusatz eines Aminosäurehydrochlorids, insbebesondere Cysteinhydrochlorid, wodurch auch noch eine zusätzliche Verstärkung der Wellwirkung erzielt werden kann, und Glycinhydrochlorid.

Es können jedoch auch weitere Aminosäurehydrochloride, beispielsweise die des Alanins, Valins, Leucins, lsoleucins, Prolins, Tryptophans, Phenylalanins, Methionins, Serins, Tyrosins, Threonins, Asparagins, Glutamins oder Histidins, enthalten sein.

Der Anteil des Aminosäurehydrochlorids wird bestimmt durch die Einstellung des erforderlichen pH-Wertes; der Mindestanteil liegt bei etwa 0,5 Gew.-%, der Höchstanteil bei etwa 5 Gew.-%, bezogen auf reine Aminosäure und die Gesamtzusammensetzung des Mittels (also die Summe der bis zur Anwendung getrennt gehaltenen Zusammensetzungen).

Bei solchen Zweikomoponenten-Präparaten weist dann die das alkalisierende Ammoniumsalz enthaltene Zusammensetzung einen alkalischen pH-Wert, vorzugsweise im Bereich zwischen etwa 8 und 9,5, auf.

Die Fixierung der durch Anwendung der erfindungsgemäßen Dauerwell-Zusammensetzungen verformten Haare erfolgt durch die ebenfalls bekannte und übliche Neutralisation mittels Wasserstoffperoxid oder Alkalibromaten.

Falls die erfindungsgemäßen Zusammensetzungen in wie bis zur Anwendung voneinander getrennten Phasen vorliegen, erfolgt die getrennte Unterbringung der beiden Zusammensetzungen bis zur Anwendung des Mittels vorzugsweise in den seit langem bekannten Zweikammerbehältern, deren Trennwand bei der Anwendung durch geeignete Mittel zerstört wird.

Solche Zweikammerbehältnisse, die sich insbesondere für die erfindungsgemäßen Zusammensetzungen eignen, sind beispielsweise in den deutschen Offenlegungsschriften 35 28 525, 36 06 003, 38 12 343 und 38 37 595 beschrieben.

Die folgenden Beispiele dienen der Illustration der Erfindung.

### Beispiel 1

### Dauerwelle für normales Haar

**Zusammensetzung A:**

| | |
|---|---|
| Ammoniumhydrogencarbonat | 4,5 (g) |
| Kationisches Polymer | 1,0 |
| Nichtionischer Lösungsvermittler (Ethylenoxid-Kondensat) | 0,8 |
| Isopropylalkohol | 1,5 |
| Diethylenglykolmonoethylether | 2,0 |
| Cocoamidopropylbetain | 1,0 |
| Trübungsmittel, Parfum | q.s. |
| Ammoniak, 25%ig | @ pH 8,4 |
| Wasser | @ 72,0 |

**Zusammensetzung B:**

| | |
|---|---|
| Ammoniumthioglykolat, 70%ig | 18,0 (g) |
| 2-Thiomilchsäure | 2,0 |
| 2-Methyl-2,4-pentandiol | 0,5 |
| Ammoniak, 25%ig | @ pH 5,5 |
| Wasser | @ 28,0 |

Die Zusammensetzungen A und B wurden getrennt in eine übliche Zweikammerdose verpackt.

Beim Zusammenbringen beider Zusammensetzungen wurde ein Produkt mit einem pH-Wert von 7,4 erhalten, das bei der Anwendung in einer üblichen Dauerwellbehandlung mit anschließender Peroxid-Fixierung eine exzellent ausgebildete, stabile Dauerwellung ohne jedwede Haarschädigung erzielte.

### Beispiel 2

### Neutraldauerwelle für gefärbtes Haar

**Zusammensetzung A:**

| | |
|---|---|
| Ammoniumhydrogencarbonat | 3,5 (g) |
| Kationisches Polymer | 0,5 |
| Nichtionischer Lösungsvermittler (Ethoxylat)) | 0,8 |
| Parfum | 0,3 |
| Ethanol | 0,5 |
| 1-Methoxypropanol(-2) | 1,0 |
| Ammoniak, 25%ig | @ pH 8,3 |
| Wasser | @ 72,0 |

**Zusammensetzung B:**

| | |
|---|---|
| Ammoniumthioglykolat, 70%ig | 13,0 (g) |
| 2-Thiomilchsäure | 0,5 |
| 1,6-Hexandiol | 1,5 |
| Ammoniak, 25%ig | @ pH 5,5 |
| Wasser | @ 28,0 |

Die Verpackung und Applikation der Zusammensetzung A und B erfolgten wie in Beispiel 1 beschrieben.

Die anwendungsfertige Gesamtmischung wies einen pH-Wert von 7,4 auf.

### Beispiel 3

### Dauerwelle für normales Haar

| | |
|---|---|
| Ammoniumhydrogencarbonat | 5,0 (g) |
| Ammoniumthioglykolat, 60%ig | 21,3 |
| 2-Methyl-2,4-pentandiol | 3,0 |
| n-Propanol | 1,5 |
| Nichtionogener Lösungsvermittler | 0,7 |
| Kationisches Polymer | 0,5 |
| Parfum | 0,3 |
| Ethoxydiglykol | 0,5 |
| Ammoniak, 25%ig | @ pH 8,3 |
| Wasser | @ 100,0 |

Auch mit dieser Zusammensetzung wurde, nach Peroxid-Fixierung, eine ausdrucksvolle Dauerwellung erhalten; auch bei mehrfach aufeinanderfolgender Anwendung war keinerlei Haarschädigung festzustellen.

### Beispiel 4

### Dauerwelle für geschädigtes Haar

| | |
|---|---|
| Ammoniumhydrogencarbonat | 2,5 (g) |
| Ammoniumthioglykolat, 60%ig | 15,0 |
| 1,5-Pentandiol | 0,5 |
| Nichtionogener Lösungsvermittler | 0,7 |
| Parfum | 0,3 |
| Ethanol | 0,5 |
| 1-Methoxypropanol(-2) | 4,0 |
| Ammoniak, 25%ig | @ pH 8,0 |
| Wasser | @ 100,0 |

Es wurde ein ähnlich gutes Dauerwellergebnis wie mit der in Beispiel 3 beschriebenen Zusammensetzung erhalten.

## Patentansprüche

1. Mittel zur dauerhaften Verformung von menschlichen Haaren, enthaltend mindestens eine reduzierend wirkende Thioverbindung und eine Kombination aus
a) 0,25 bis 7,5 Gew.-% Ethylalkohol, n-Propylalkohol und/oder Isopropylalkohol;
b) 0,25 bis 7,5 Gew.-% mindestens einer Komponente aus der Gruppe Pentandiole, Hexandiole und/oder Methylpentandiole und
c) 0,25 bis 17,5 Gew.-% 1-Methoxypropanol(-2), 1-Ethoxypropandiol(-2) und/oder Diethylenglykolmonomethyl bzw. -monoethylether,
jeweils berechnet auf die Gesamtzusammensetzung des gebrauchsfertigen Mittels.

2. Mittel nach Anspruch 1, **dadurch gekennzeichnet, daß** die gebrauchsfertige, zum Aufbringen auf das Haar bestimmte Zusammensetzung einen pH-Wert zwischen 7 und 9,5 aufweist.

3. Mittel nach Anspruch 1 oder 2, **dadurch gekennzeichnet, daß** es als reduzierend wirkende Thioverbindung Thioglykolsäure und/oder 2-Thiomilchsäure bzw. deren Salze enthält.

4. Mittel nach einem oder mehreren der vorhergehenden Ansprüche, **dadurch gekennzeichnet, daß** es als Komponente b) 1,2-Pentandiol, 1,5-Pentandiol, 1,6-Hexandiol und/oder 2-Methyl-2,4-pentandiol in einer Menge von 0,5 bis 5 Gew.-%, berechnet auf die Gesamtzusammensetzung, enthält.

5. Mittel nach einem oder mehreren der vorhergehenden Ansprüche, **dadurch gekennzeichnet, daß** es als Bestandteil c) 1 bis 5 Gew.-% Diethylenglykolmonothylether enthält.

6. Mittel nach einem oder mehreren der vorhergehenden Ansprüche, **dadurch gekennzeichnet, daß** es 0,5 bis 5 Gew.-% Ammoniumhydrogencarbonat, Ammoniumcarbonat und/oder Ammoniumcarbamat enthält.

7. Mittel nach einem oder mehreren der vorhergehenden Ansprüche, **dadurch gekennzeichnet, daß** es aus zwei bis zur Anwendung voneinander getrennt gehaltenen Zusammensetzungen besteht, wobei die eine Zusammensetzung (A) mindestens ein Aminosäuresalz in wäßrigem Medium enthält und auf einen pH-Wert zwischen 4,5 und 6,5 eingestellt ist, und die zweite, davon getrennt gehaltene Zusammensetzung (b) Ammoniumhydrogencarbonat, Ammoniumcarbonat und/oder Ammoniumcarbamat enthält und einen pH-Wert zwischen 8 und 9,5 aufweist, und beide Zusammensetzungen unmittelbar vor der Anwendung auf menschliches Haar vermischt werden und die **dadurch** hergestellte gebrauchsfertige Zusammensetzung (AB) einen pH-Wert zwischen 7 und 8 aufweist.

8. Mittel nach Anspruch 7, **dadurch gekennzeichnet, daß** die reduzierende Thioverbindung zumindest teilweise in der Zusammensetzung (A) enthalten ist.

## Claims

1. Composition for the permanent waving of human hair, comprising at least one thio compound with a reducing effect and a combination of
a) 0.25 % to 7.5 % by weight of ethyl alcohol, n-propyl alcohol and/or isopropyl alcohol;
b) 0.25 % to 7.5 % by weight of at least one component from the group pentanediols, hexanediols and/or methyl pentanediols; and
c) 0.25 % to 7.5 % by weight of 1-methoxypropanol(-2), 1-ethoxy-propanol(-2) and/or diethyleneglycol monomethyl or monomethyl ether, each calculated to the total ready-to-use composition.

2. Composition according to claim 1, wherein the ready-to-use composition destined for application onto the hair has a pH-value between 7 and 9.5.

3. Composition according to claims 1 or 2, **characterized in that** it contains thioglycolic acid and/or 2-thiolactic acid or the salts thereof as thio compound having a reducing effect.

4. Composition according to one or more of the above claims, **characterized in that** as component b) it contains 1.2-pentanediol, 1.5-pentanediol, 1.6-hexanediol and/or 2-methyl-2.4-pentanediol in an amount of 0.5 to 5 % by weight, calculated to the total composition.

5. Composition according to one or more of the above claims, **characterized in that** as component c) it contains 1 % to 5 % by weight, calculated to the total compostion of diethyleneglycol monoethyl ether.

6. Composition according to one or more of the above claims, **characterized in that** it contains 0.5 % to 5 % by weight, calculated to the total composition, of ammonium hydrogen carbonate, ammonium carbonate and/or ammonium carbamate.

7. Composition according to one or more of the above claims, **characterized in that** it consists of two compositions kept separately until their application, whereby the one composition (A) contains at least one amino acid salt in aqueous medium and has a pH-value adjusted between 4.5 and 6.5, and the second composition (B), kept separately therefrom, contains ammonium hydrogen carbonate, ammonium carbonate and/or ammonium carbamate and shows a pH-value between 8 and 9.5, and both compositions are admixed immediately prior to application onto human hair, and the ready-to-use composition (AB) thus prepared has a pH-value between 7 and 8.

8. Composition according to claim 7, wherein the reducing thio compound is at least partially contained in the composition (A).

## Revendications

1. Agent de mise en forme durable des cheveux humains, qui contient au moins un composé thio à action réductrice et une combinaison constituée de :
a) 0,25 à 7,5 % en poids d'alcool éthylique, d'alcool n-propylique et/ou d'alcool isopropylique,
b) 0,25 à 7,5 % en poids d'au moins un composant sélectionné dans le groupe des pentane diols, des hexane diols et/ou des méthylpentane diols et
c) 0,25 à 7,5 % en poids de 1-méthoxypropanol(-2), de 1-éthoxypropane diol(-2) et/ou de monométhyléther ou de monoéthyléther de diéthylène glycol,
tous calculés par rapport à la composition totale de l'agent prêt à l'emploi.

2. Agent selon la revendication 1, **caractérisé en ce que** la composition prête à l'emploi destinée à être appliquée sur les cheveux a un pH dont la valeur est comprise entre 7 et 8,5.

3. Agent selon les revendications 1 ou 2, **caractérisé en ce que** comme composé thio à action réductrice, il contient de l'acide thioglycolique et/ou de l'acide 2-thiolactique ou leurs sels.

4. Agent selon l'une ou plusieurs des revendications précédentes, **caractérisé en ce que** comme composant b), il contient du 1,2-pentane diol, du 1,5-pentane diol, du 1,6-hexane diol et/ou du 2-méthyl-2,4-pentane diol en quantité de 0,5 à 5 % en poids par rapport à la composition totale.

5. Agent selon l'une ou plusieurs des revendications précédentes, **caractérisé en ce que** comme composant c), il contient de 1 à 5 % en poids de monoéthyléther de diéthylène glycol.

6. Agent selon l'une ou plusieurs des revendications précédentes, **caractérisé en ce qu'**il contient de 0,5 à 5 % en poids d'hydrogénocarbonate d'ammonium, de carbonate d'ammonium et/ou de carbamate d'ammonium.

7. Agent selon l'une ou plusieurs des revendications précédentes, **caractérisé en ce qu'**il est constitué de deux compositions maintenues séparées l'une de l'autre jusqu'à leur application, l'une (A) des compositions contenant au moins un sel d'acide aminé dans un milieu aqueux dont le pH est ajusté à une valeur comprise entre 4,5 et 6,5 et la deuxième composition (b) séparée d'elle contenant de l'hydrogénocarbonate d'ammonium, du carbonate d'ammonium et/ou du carbamate d'ammonium et ayant un pH d'une valeur comprise entre 8 et 9,5, les deux compositions étant mélangées immédiatement avant d'être appliquées sur les cheveux humains et la composition (AB) prête à l'emploi ainsi préparée présentant un pH d'une valeur comprise entre 7 et 8.

8. Agent selon la revendication 7, **caractérisé en ce que** le composé thio à action réductrice est contenu au moins en partie dans la composition (A).
